# EUROPÄISCHE PATENTSCHRIFT

(11) **EP 2 921 143 B1**
(45) Veröffentlichungstag und Bekanntmachung des Hinweises auf die Patenterteilung: **05.05.2021**
(21) Anmeldenummer: 15157738.4
(22) Anmeldetag: 05.03.2015
(51) Int. Cl.: A61B 17/88

(54) **STEMPELINSTRUMENT SOWIE DIESES UMFASSENDES SYSTEM ZUR BEHANDLUNG EINER KNOCHEN- ODER KNORPELSTRUKTUR**
STAMP INSTRUMENT AND SYSTEM COMPRISING SAME FOR TREATING A BONE OR CARTILAGINOUS STRUCTURES
INSTRUMENT DE TAMPON ET SYSTÈME COMPRENANT CELUI-CI DESTINÉ À TRAITER UNE STRUCTURE OSSEUSE OU DE CARTILAGE

(30) Priorität: 17.03.2014 DE 202014101197 U
(43) Veröffentlichungstag der Anmeldung: 23.09.2015
(73) Patentinhaber: Joline GmbH & Co. KG, 72379 Hechingen (DE)
(72) Erfinder: HOLDER, Bernd, 72379 Hechingen (DE); STEEGERS, Anselm, 72379 Hechingen (DE); AMMERICH, Uwe, 72379 Hechingen (DE)
(74) Vertreter: Witte, Weller & Partner Patentanwälte mbB

(56) Entgegenhaltungen:
- US-A- 4 769 011
- US-A- 5 665 093
- US-A1- 2004 068 264
- US-A1- 2006 064 101
- US-B1- 7 963 967

## Beschreibung

Die vorliegende Erfindung betrifft ein Stempelinstrument zum Vorwärtsbewegen durch eine Kanüle und zum Vorwärtsbewegen eines in der Kanüle vorliegenden Materials in eine Kavität einer Knochen- oder Knorpelstruktur, insbesondere eines Wirbelkörpers, wobei das Stempelinstrument einen Schaft, ein erstes proximales Ende und ein zweites distales Ende aufweist, sowie einen am ersten proximalen Ende vorgesehenen Griff, sowie Systeme zur Behandlung von Knochen- oder Knorpelstrukturen, insbesondere von Wirbelkörpern, die ein solches Stempelinstrument aufweisen.

Derartige Stempelinstrumente und Systeme zur Behandlung von Knochen- bzw. Knorpelstrukturen werden im Stand der Technik bspw. bei der Behandlung Wirbelkörpern bekannt, und kommen insbesondere bei der Behandlung von Wirbelbrüchen zum Einsatz.

Wirbelbrüche sind insbesondere osteoporotisch, traumatisch oder tumorös bedingt, und werden gegenwärtig durch die Verfahren der Vertrebroplastie oder Kyphoplastie behandelt.

Während bei der Vertebroplastie eine Hohlnadel durch die Bogenwurzeln des betroffenen Wirbels eingebracht und anschließend Knochenzement in den zu behandelnden Wirbelkörper eingespritzt wird, der in kurzer Zeit aushärtet, wird bei dem ebenfalls minimalinvasiven Verfahren der Kyphoplastie zunächst mittels einer Kanüle ein kollabierter Ballon in den zu behandelnden Wirbelkörper eingeführt. Durch Inflatieren/Expandieren des Ballons kann der zusammengebrochene Wirbel teilweise wieder aufgerichtet und anschließend fixiert werden, indem in die entstandene Kavität(en) Knochenzement eingespritzt oder anderweitig eingeführt wird, der innerhalb weniger Minuten aushärtet und damit den gebrochenen Wirbel stabilisiert.

Je nach Technik werden kleinere oder größere Zugänge zum Wirbelkörper benötigt. Neuere Techniken benötigen leidglich kleinere Zugänge und legen nur sehr kleine Kavitäten bzw. Gänge an. Dadurch kann der eingeführte Knochenzement gleichmäßig verteilt und die gesunde Spongiosa mit dem Knochenzement verzahnt werden.

Die hierfür notwendigen Instrumente umfassen dabei regelmäßig zumindest eine Zugangs- bzw. Arbeitskanüle, mit der der perkutane Zugang zum Wirbelkörper bereitgestellt wird, eine Baugruppe aus Knochenzement-Kanüle und Stempel, mit welchem der in der Knochenzement-Kanüle vorliegende Knochenzement aus dieser in den Wirbelkörper vorgeschoben werden kann, sowie ggf. ein Instrument zum Ausbilden einer Kavität im Wirbelkörper, bspw. einem Ballonkatheter. Die Einführung der Instrumente in den Wirbelkörper erfolgt dabei regelmäßig unter fluoroskopischer/röntgonologischer Kontrolle.

So sind bspw. aus der US 7963967 B1 und WO 00/09024 A1 solche Systeme bekannt, das eine solche Zugangskanüle, ein eine Kavität ausbildendes Instrument und eine Baugruppe aus Kanüle und Stopfer umfasst.

Die im Stand der Technik bekannten Systeme haben dabei u.a. den Nachteil, dass es durch die verschiedenen, eher sperrigen Instrumente schwer ist, diese, den Patienten und das Röntgengerät zur Überwachung des Verfahrens gleichzeitig und einfach zu handhaben.

Aufgabe der vorliegenden Erfindung ist es daher ein System bereit zu stellen, das eine einfache und kompakte bzw. flexiblere Handhabung ermöglicht, wobei gleichzeitig die Funktionalität der einzusetzenden Instrumente uneingeschränkt erhalten bleibt.

Erfindungsgemäß wird diese Aufgabe gelöst durch ein Stempelinstrument zum Vorwärtsbewegen durch eine Kanüle und zum Vorwärtsbewegen eines in der Kanüle vorliegenden Materials in einer Kavität eines Wirbelkörpers, wobei das Stempelinstrument einen Schaft, ein erstes proximales Ende und ein zweites distales Ende aufweist, sowie einen am ersten proximalen Ende vorgesehenen Griff; das Stempelinstrument weist ferner einen flexiblen Schaft mit Markierungen auf, sowie an seinem zweiten, distalen Ende ein starres Stempelelement, das mit dem flexiblen Schaft verbunden ist.

Die der Erfindung zugrunde liegende Aufgabe wird ferner gelöst durch ein System zur Behandlung von Wirbelkörpern, das zumindest das erfindungsgemäße Stempelinstrument aufweist, sowie eine erste Kanüle zum Vorlegen von Knochenzement, welche Kanüle mit dem Stempelinstrument eine Baugruppe bildet.

Die der Erfindung zugrunde liegende Aufgabe wird auf diese Weise vollkommen gelöst. So wird mit der flexiblen Ausbildung des Schafts, in Kombination mit dem Stempelelement am distalen Ende zum einen sicher gestellt, dass das Stempelinstrument nach wie vor zuverlässig zum Vorschieben von Material in eine Knochen- oder Knorpelstruktur eingesetzt werden kann, wobei aber gleichzeitig der Schaft selber gebogen werden kann. Dies ist insbesondere dann von Vorteil, wenn bei einem Kyphoplastie-Eingriff bspw. eine Arbeitskanüle am Patienten an- bzw. eingesetzt ist, hierin dann eine weitere Kanüle eingeführt ist, über welche der Knochenzement zugeführt wird, und dann hierauf das Stempelinstrument aufgesetzt wird: Durch seine flexible Ausgestaltung kann der Schaft dann, wenn das Stempelinstrument erst mit einem kleinen Abschnitt am distalen Ende in die erste Kanüle eingeschoben wird, und sich somit der Großteil des Stempelinstruments noch außerhalb der Kanüle befindet, gebogen werden, so dass er nicht die initiale Bauhöhe des Systems weiter verlängert. Ein Röntgen-Kontrollgerät kann dadurch einfacher über den Patienten platziert werden. Gleichzeitig wird mit dem starren Stempelelement am distalen Ende sichergestellt, dass der Knochenzement wirksam vorgeschoben werden kann.

Vorliegend, wie auch allgemein im betreffenden Gebiet, wird unter dem proximalen Ende dasjenige Ende des zwei Enden aufweisenden Stempelinstruments (oder überhaupt eines zwei Enden aufweisenden medizinischen Instruments) verstanden, das näher am Handhabenden liegt. Das distale Ende ist entsprechend dasjenige Ende, das weiter vom Handhabenden entfernt ist als das andere, das proximale Ende.

Ferner wird unter "flexibel" vorliegend eine reversibel biegsame Ausbildung des Schafts des Stempelinstruments verstanden, aufgrund dessen der Schaft in verschiedene, dem Anwender beliebige Richtung gebogen und zurückgebogen werden kann.

"Starr" bedeutet vorliegend hingegen, dass das betreffende Element nicht oder nur unter Aufbringung von extremen, unter normalen Verfahrensbedingungen nicht aufzubringenden Kräften verbogen werden kann. Gemäß einer bevorzugten Ausführungsform ist das Stempelelement aus massivem Edelstahl gebildet.

Während also der Schaft flexibel und biegsam ausgebildet ist, und damit gegenüber einer entgegenwirkenden Kraft weich bzw. eher weich ist, ist das distale Ende mit dem Stempelelement starr bzw. fest und in Bezug auf das einzusetzende Material, bspw. Edelstahl, massiv ausgebildet.

Vorliegend wird das erfindungsgemäße Stempelinstrument bzw. System überwiegend in Bezug auf die Behandlung von Wirbelkörpern beschrieben, es versteht sich, dass aber auch jede andere Knochen- oder Knorpelstruktur mit dem erfindungsgemäßen System behandelt werden kann.

Gemäß einer bevorzugten Ausführungsform des erfindungsgemäßen Stempelinstruments ist das Stempelelement mit dem flexiblen Schaft verschweißt.

Gemäß einer weiteren Ausführungsform weisen der Schaft und/oder das Stempelelement jeweils ein Material auf oder bestehen insgesamt aus diesem, welches ausgewählt ist aus der Gruppe umfassend Metalle und nicht-metallische Werkstoffe, insbesondere solche, die für medizintechnische Anwendungen geeignet sind, und insbesondere Edelstahl oder Kunststoff.

Bei dem erfindungsgemäßen Stempelinstrument ist ferner bevorzugt, wenn der Schaft in den Griff durch eine geeignete Fügetechnik, bspw. durch Verkleben oder Umspritzen eingebracht ist.

Es versteht sich hierbei, dass der Griff für diese Zwecke eine Bohrung aufweisen kann, die in ihrem Durchmesser dem Durchmesser des Schaftes angepasst ist, so dass dieser über sein proximales Ende in der Bohrung aufgenommen werden kann. Für die Verklebung des Schafts im Griff kommen verschiedene, im Stand der Technik verfügbare Klebemittel in Betracht, und sind dem Fachmann geläufig. Auch Techniken zur Umspritzung des Schafts für eine Verbindung mit dem Griff sind dem Fachmann bekannt.

Gemäß einer Ausführungsform des erfindungsgemäßen Stempelinstruments weist der Schaft einen sich über die Länge des Schafts erstreckenden Edelstahl-Kern sowie eine diesen umwickelnden Edelstahl-Draht auf.

Der Edelstahlkern ist ein massiver, eher dünner Stab mit Durchmessern zwischen ca. 0,2 mm und ca. 3 mm aus Edelstahl, und bietet die Basis für eine gewisse Stabilität. Dieser Edelstahlkern ist erfindungsgemäß über seine Längsachse bzw. die des Schafts mit einem Edelstahldraht umwickelt; der Edelstahldraht hat dabei einen Durchmesser von zwischen ca. 0,1 mm und ca. 1 mm. Die Kombination aus dem Edelstahlkern und dem diesen umwickelnden Edelstahldraht begründet die Flexibilität des Schafts des erfindungsgemäßen Stempelinstruments. Dabei wird mit zunehmenden Durchmesser des Edelstahlkerns die Steifigkeit des Schafts höher, bzw. die Flexibilität niedriger.

Gemäß einer bevorzugten Ausführungsform weist der Schaft des erfindungsgemäßen Stempelinstruments eine Biegesteifigkeit von zwischen ca. 1700 N*mm² und ca. 27200 N*mm² auf. Die Biegesteifigkeit wird dabei als Produkt aus Elastitätsmodul (E) und Flächenträgheitsmoment (I) verstanden bzw. ist entsprechend definiert.

Dabei ist gemäß einer bevorzugten Ausführungsform der Edelstahldraht über die gesamte Länge des Edelstahlkerns gewickelt. Es versteht sich dabei, dass selbstverständlich auch Wickelungen vorgesehen sein können, die sich nur teilweise über die Länge des Edelstahlkerns erstrecken: insbesondere können Ausführungen vorgesehen sein, bei welchen der Edelstahldraht ausschließlich über einen proximalen oder distalen oder einen mittigen Bereich des Edelstahlkerns gewickelt ist.

Das Stempelinstrument besitzt vorteilhafterweise eine Länge von zwischen ca. 10 cm bis ca. 50 cm.

Gemäß einer bevorzugten Ausführung des erfindungsgemäßen Stempelinstruments weist der Schaft Markierungen auf, die über seine Längsachse auf seinem äußeren Umfang verteilt sind.

Diese Markierungen haben den Vorteil, dass anhand dieser der Handhabende beim vorantreiben durch eine Kanüle bestimmen kann, wie weit das Stempelinstrument vorgeschoben worden ist bzw. wird. Auch kann mittels der am Schaft vorgesehenen Markierungen ggf. bestimmt werden, welche Menge an Knochenzement in die Kavität geschoben wurde, und welche Menge noch in der Kanüle vorliegt, nachdem das Stempelinstrument abschnittsweise durch die Kanüle vorgeschoben wurde.

Wie weiter oben erwähnt betrifft die vorliegende Erfindung auch ein System zur Behandlung von menschlichen Wirbelkörpern, zumindest umfassend das erfindungsgemäße Stempelinstrument, sowie eine erste Kanüle zum Zuführen eines im Wirbelkörper aushärtbaren Materials zu einer in dem Wirbelkörper gebildeten Kavität, wobei das Stempelinstrument und die Kanüle derart ausgebildet und dimensioniert sind, dass das Stempelinstrument durch die Kanüle vorwärts bewegt werden kann, um in der Kanüle vorliegendes Material in die Kavität des Wirbelkörpers zu bewegen.

Das erfindungsgemäße System bietet den Vorteil, dass Stempelinstrument und die erste Kanüle derart zusammenarbeiten, dass mittels des Stempelinstruments Material, insbesondere Knochenzement, aus der ersten Kanüle herausgetrieben, also stempelartig vorgeschoben werden kann. Die Kanüle, die ggf. einen Kanülengriff aufweisen kann, ist dabei vorzugsweise aus einem steifen bzw. harten und nicht-flexiblen Material, und besitzt eine Länge, die kürzer ist als die Länge des Stempelinstruments inklusive Griff.

Wird die erste Kanüle, die mit dem in eine Kavität einzuführenden Knochenzement gefüllt wird bzw. ist, und ein erstes proximales Kanülenende und ein zweites distales Kanülenende besitzt, mit ihrem distalen Kanülenende über bzw. zu der Kavität in dem Wirbelkörper geführt, so kann das Stempelinstrument am proximalen Kanülenende in die Kanüle eingeführt und nach distal vorgeschoben werden, wodurch auch der in der Kanüle befindliche Knochenzement in Richtung distal, d.h. in die Kavität vorgeschoben werden kann.

Bei Vorliegen von Markierungen an dem Schaft des erfindungsgemäßen Stempelinstruments kann vorteilhafterweise die Vorschubtiefe in die erste Kanüle von außerhalb der Kanüle bestimmt werden.

Aufgrund der Flexibilität des Schaftes kann dieser darüber hinaus mit seinem noch nicht in die erste Kanüle eingeführten Abschnitt gebogen werden, so dass sich der Aufbau aus Kanüle und aufgesetztem Stempelinstrument nicht stark in die Längsachse verlängert, und dadurch auch nicht die Platzierung anderer Gerätschaften über der ersten Kanüle bzw. dem Patienten behindern. Das Stempelelement und ggf. weitere kurze - und mit weiterem Vorschieben länger werdende - Abschnitte befinden sich dabei in der ersten Kanüle. Dadurch kann das Vorschieben von bspw. Knochenzement bei jedem Schritt bspw. röntgenologisch durch ein Röntgengerät, das über den Patienten bzw. über dem zu behandelnden Wirbelkörper angebracht wird, kontrolliert werden, ohne dass die Bauhöhe des Systems dieser Kontrolle entgegensteht.

Gemäß einer weiteren Ausführungsform des erfindungsgemäßen Systems weist die erste Kanüle einen röhrchenförmigen hohlen Körper mit einer Längsachse, einem distalen und einem proximalen offenen Kanülenende auf, sowie einen einen Durchgang aufweisenden Kanülengriff am proximalen Kanülenende.

Mittels des Griffs am proximalen Kanülenende kann die erste Kanüle gut gegriffen werden, wobei gleichzeitig durch den Durchgang bzw. die Bohrung im Kanülengriff sicher gestellt ist, dass das Stempelinstrument einfach in die Kanüle eingeführt werden kann.

Gemäß einer weiteren Ausführungsform weist die erste Kanüle Markierungen über ihre Längsachse auf, wobei die Markierungen - wie im Übrigen auch bei dem Stempelinstrument - partiell oder über die gesamte Kanülenlänge vorgesehen sein können.

Gemäß einer weiteren Ausführungsform ist bevorzugt, wenn das System gemäß der vorliegenden Erfindung ferner eine Arbeitskanüle zum Bereitstellen eines Zugangs zu einem Wirbelkörper aufweist, wobei die Arbeitskanüle derart ausgebildet und dimensioniert ist, dass die erste Kanüle in die Arbeitskanüle einführbar ist.

Mit der Arbeitskanüle wird ein Zugang bzw. Arbeitskanal zu dem zu behandelnden Wirbelkörper geschaffen, über welchen weitere Instrumente, und über diese schließlich der Knochenzement zugeführt werden kann.

Ferner weist das erfindungsgemäße System gemäß einer weiteren bevorzugten Ausführungsform ein Instrument zum Ausbilden einer Kavität in einem Wirbelkörper auf, welches derart ausgebildet und dimensioniert ist, dass es durch die Arbeitskanüle einführbar ist.

Das Instrument zum Ausbilden einer Kavität kann erfindungsgemäß in die Arbeitskanüle eingeführt werden, was ebenso wie alle anderen Vorgänge röntgenologisch kontrolliert werden kann. Entsprechend verfügt das Instrument über Dimensionen, die es ermöglichen, das Instrument in die Arbeitskanüle und bis zum Wirbelkörper vorzuschieben; am proximalen Ende kann das Instrument dann betätigt werden, so dass am distalen Ende, das sich im Wirbelkörper befindet, eine Kavität gebildet wird. Daher weist das Instrument auch eine Länge auf, die größer ist als die der Arbeitskanüle, da das Instrument ansonsten nicht hindurch reichen würde.

Das Instrument zum Ausbilden einer Kavität kann bspw. ein Ballonkatheter sein, der im Wirbelkörper zur Expansion gebracht werden kann, um eine Kavität zu schaffen. Alternativ kann auch ein Instrument eingesetzt werden, mittels dessen kleine Kanäle im Wirbelkörper geschaffen werden.

Erfindungsgemäß wird unter einer "Kavität" daher jeder Hohlraum in einem Wirbelkörper verstanden, der mittels des Instruments gebildet wird, und unabhängig von der Form der Kavität/des Hohlraums, die rund, oval, kanalförmig, etc. sein kann.

Weitere Vorteile und Merkmale ergeben sich aus der nachfolgenden Beschreibung und der beigefügten Zeichnung.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ein Ausführungsbeispiel der Erfindung ist in der Zeichnung dargestellt und wird mit Bezug auf diese hiernach näher beschrieben. Es zeigen:

- Fig. 1: eine schematische Darstellung einer Ausführungsform eines erfindungsgemäßen Stempelinstruments, im ungebogenen Zustand **(A)** und im gebogenen Zustand **(B);** und
- Fig. 2: eine schematische Darstellung einer Ausführungsform eines erfindungsgemäßen Systems zur Behandlung von menschlichen Wirbelkörpern, welches System die in Fig. 1 gezeigte Ausführungsform des erfindungsgemäßen Stempelinstruments, teilweise eingeführt in eine erste Kanüle, umfasst, und zwar einmal im ungebogenen Zustand **(A),** sowie einmal im gebogenen Zustand **(B).**

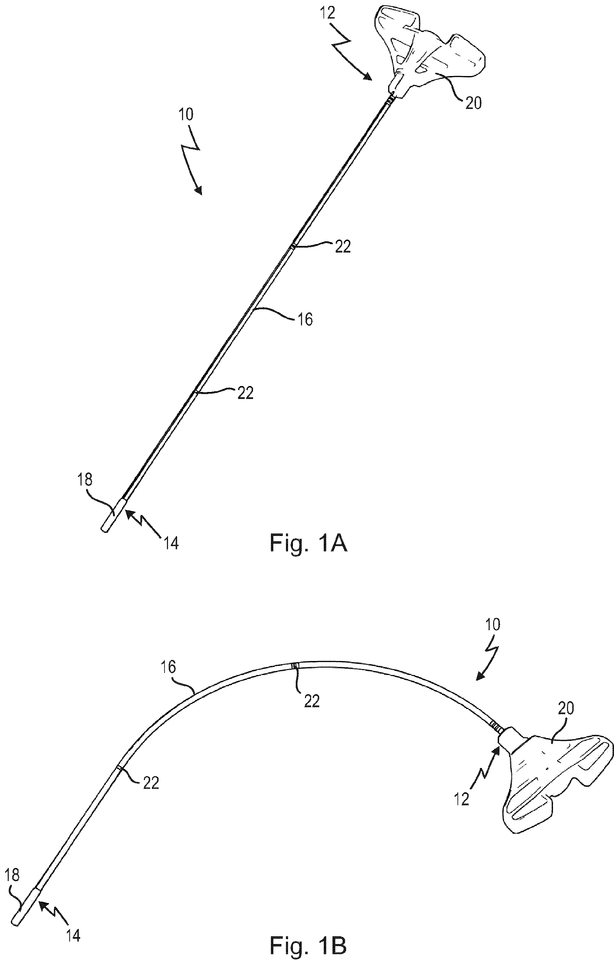

In Fig. 1A und 1B ist mit 10 insgesamt ein Stempelinstrument bezeichnet, das über eine Längsachse 11, sowie ein erstes, proximales Ende 12, sowie ein zweites distales Ende 14 aufweist. Ferner umfasst das Stempelinstrument 10 einen Schaft 16, und ein Stempelelement 18, das am distalen Ende 14 des Stempelinstruments 10 mit dem Schaft 16 verbunden ist. Der Schaft 16 selber weist ein proximales Schaftende 17 und ein distales Schaftende 18 auf.

Wie Fig. 1A und 1B zu entnehmen ist, befindet sich am distalen Ende 14 des Stempelinstruments 10 ferner ein Griff 20, der am distalen Schaftende 17 mit dem Schaft 16 fest verbunden ist. Der Griff 20 kann zum Verbinden des Schafts 16 entweder eine Bohrung aufweisen, in die der Schaft 16 eingebracht und in dieser Verklebt wird, oder der Schaft 16 wird durch Umspritzen mit dem Griff 20 verbunden.

Über die Längsachse 11 des Stempelinstruments 10, bzw. über den Schaft 16 verteilt sind Markierungen 22 vorgesehen, anhand derer der Handhabende erkennen kann, wie tief der Stempel in eine Kanüle vorgeschoben wurde, und ggf. wie viel an Material sich noch in der Kanüle befindet, durch die das Stempelinstrument 10 vorgeschoben wird, bzw. wie viel Material dadurch in den Wirbelkörper eingebracht wurde.

Fig. 1 ist ferner zu entnehmen, dass Markierungen 22 am Schaft 16 angebracht sind, über welche die Eindringtiefe des Stempelinstruments und/oder das Volumen des eingeschobenen Materials bei der Operation bemessen werden kann.

Das Stempelelement 18 ist dabei insgesamt aus Edelstahl gebildet, und daher "massiv". Der Schaft 16 ist vorzugsweise zweiteilig aufgebaut, nämlich aus einem Edelstahlkern mit einem diesen umwickelnden Edelstahldraht. Diese Konstruktion bietet den Vorteil, dass das Stempelinstrument im Bereich des Schafts 10 flexibel ist, d.h. von der Längsachse weggebogen werden kann, was in Fig. 1B gezeigt ist: Hier ist der Schaft 16 des Stempelinstruments gebogen - das Stempelelement aufgrund seiner massiven Bauweise nicht -wodurch bei einem Aufsetzen und teilweise Einführen in eine Kanüle die Gesamt-Bauhöhe der Anordnung aus Stempelinstrument und Kanüle verringert werden kann. Im Bereich des Stempelelements 18 ist das Stempelinstrument 10 nicht flexibel, so dass dieses ein effizientes Vorschieben des in einer Kanüle vorliegenden Materials erreichen kann.

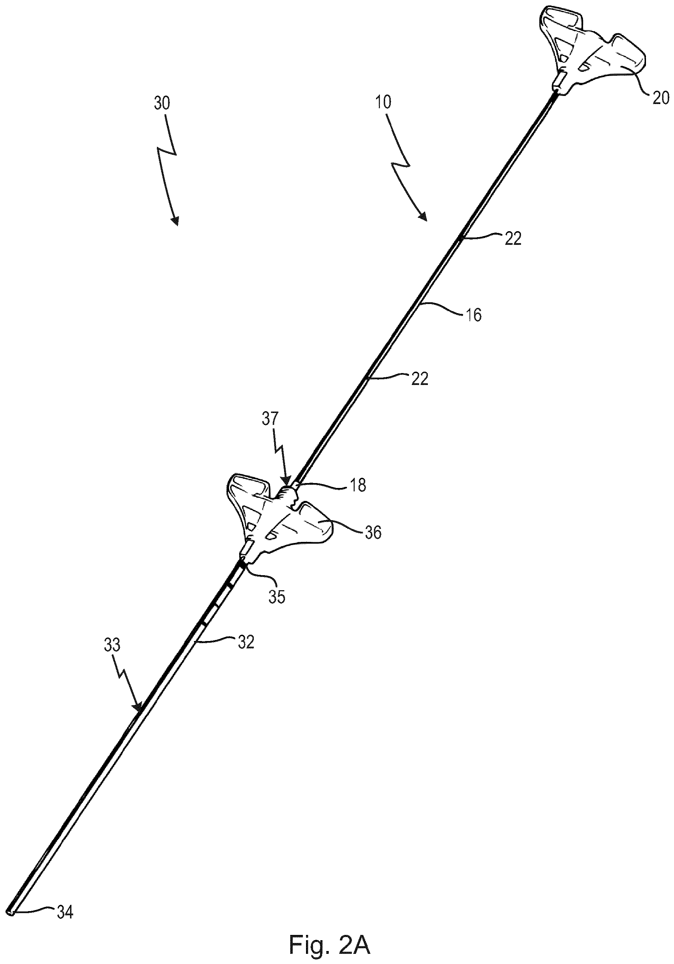

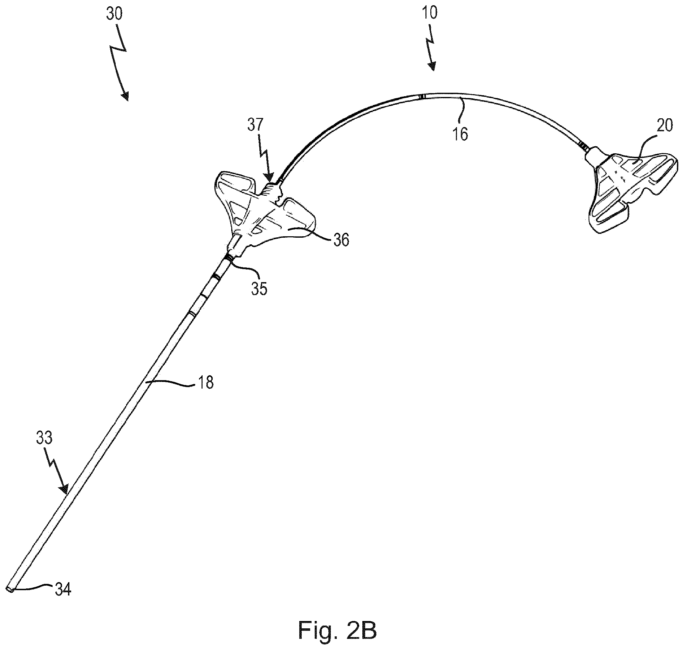

In Fig. 2A und 2B ist eine Ausführungsform eines erfindungsgemäßen Systems 30 gezeigt, das das erfindungsgemäße Stempelinstrument 10 sowie eine erste Kanüle 32 umfasst. Die erste Kanüle 32 arbeitet mit dem erfindungsgemäßen Stempelinstrument 10 derart zusammen, dass das Stempelinstrument durch die erste Kanüle 32 führbar ist, und Material, bspw. Knochenzement, das in der ersten Kanüle 32 vorliegt, in den Wirbelkörper vorschieben kann. Hierzu ist die erste Kanüle 32 derart dimensioniert, dass in diese das Stempelinstrument 10 eingeführt werden kann, und zwar derart, dass einerseits eine Reibung an den Innenwänden der Kanüle 32 möglichst gering gehalten wird, während andererseits gleichzeitig sicher gestellt ist, dass möglichst alles in der Kanüle 32 vorliegende Material in den Wirbelkörper vorgeschoben werden kann. Stempelinstrument 10 und Kanüle 32 arbeiten daher nach dem Prinzip Zylinder/Kolben, bzw. Spritze zusammen.

Als Material, das in den Wirbelkörper vorgeschoben wird, kommt jedes im Stand der Technik bekannte Material, insbesondere Knochenzement, in Betracht. Die Menge und konkrete Beschaffenheit wird insbesondere von dem jeweils zu behandelnden Patienten und der Erkrankung des Wirbelkörpers anhängen, und es wird dem Fachmann ausgehend von der vorliegenden Offenbarung und seinem allgemeinen Fachwissen offensichtlich sein, welche Mengen und welche Zusammensetzungen für das Material jeweils geeignet sind.

Die erste Kanüle 32 weist einen röhrchenförmigen hohlen Körper 33 auf, mit einem distalen Kanülenende 34 und einem proximalen Kanülenende 35. Ferner ist am proximalen Kanülenende 35, also dem Ende das näher zum Handhabenden liegt als das andere, distale Ende 34, ein Kanülengriff 36 vorgesehen. Dieser weist einen Durchgang 37 bzw. eine Bohrung auf, über welche das Kanüleninnere zugänglich ist, und durch welchen das Stempelinstrument 10 in die erste Kanüle 32 eingeführt werden kann.

Das Stempelinstrument 10 weist dabei eine Länge auf, die vorzugsweise derart ist, dass das distale Ende 14 des Stempelinstruments 10 im Zustand, in welchem das Stempelinstrument in die erste Kanüle 32 eingeführt ist, mit dem distalen Kanülenende 34 endet. Dadurch wird sichergestellt, dass alles, in der Kanüle 32 vorliegende Material aus dieser hinaus und in den Wirbelkörper vor geschoben werden kann.

In Fig. 2A ist das teilweise in die erste Kanüle 32 eingeführte erfindungsgemäße Stempelinstrument 10 gezeigt, wobei sich das Stempelinstrument 10 hier im (noch) ungebogenen Zustand befindet, und in Fig. 2B in einem flexibel gebogen wurde. Durch den Vergleich der Fig. 2A und 2B ist zu erkennen, dass durch das seitliche, d.h. weg von der Längsachse, Biegen die Gesamtbauhöhe der Anordnung aus Kanüle 32 und Stempelinstrument 10 deutlich verringert werden kann.

Trotz des gebogenen, flexiblen Zustands kann das Stempelinstrument und mit diesem das Material in der Kanüle 32 vorwärts, d.h. nach distal (weg vom Handhabenden und in Richtung des Wirbelkörpers) geschoben werden, was insbesondere auf das massive Stempelelement 18 und den flexiblen Aufbau des Schafts 16 zurückzuführen ist.

Das in Fig. 2 gezeigte erfindungsgemäße System aus Kanüle 32 und flexiblem Stempelinstrument 10 kann ferner noch eine Arbeitskanüle umfassen, welche einen Zugang zu einem Wirbelkörper bereitstellt und welche einen Kanal zum Einführen weiterer Arbeitsinstrumente bietet: so können bspw. noch ein Instrument zum Schaffen einer Kavität (Kanäle oder sonstige Hohlräume) in dem Wirbelkörper durch die Arbeitskanüle vorgeschoben und betätigt werden.

## Patentansprüche

1. Stempelinstrument (10) zum Vorwärtsbewegen durch eine Kanüle und zum Vorwärtsbewegen eines in der Kanüle vorliegenden Materials in eine Kavität einer Knochen- oder Knorpelstruktur, wobei das Stempelinstrument (10) einen Schaft (16), ein erstes proximales Ende (12) und ein zweites distales Ende (14) aufweist, sowie einen am ersten proximalen Ende (12) vorgesehenen Griff (20), wobei der Schaft (16) flexibel ausgebildet ist, und **dadurch gekennzeichnet, dass** das Stempelinstrument (10) an seinem zweiten, distalen Ende (14) ein starres Stempelelement (18) aufweist, das mit dem flexiblen Schaft (16) verbunden ist.

2. Stempelinstrument (10) nach Anspruch 1, **dadurch gekennzeichnet, dass** das Stempelelement (18) mit dem flexiblen Schaft (16) verschweißt ist.

3. Stempelinstrument (10) nach Anspruch 1 oder 2, **dadurch gekennzeichnet, dass** der Schaft (16) und/oder das Stempelelement (18) jeweils ein Material aufweisen oder aus diesem bestehen, welches ausgewählt ist aus der Gruppe umfassend Metalle und nicht-metallische Werkstoffe.

4. Stempelinstrument (10) nach einem der Ansprüche 1 bis 3, **dadurch gekennzeichnet, dass** der Schaft (16) und/oder das Stempelelement (18) jeweils ein Material aufweisen oder aus diesem bestehen, welches ausgewählt ist aus Edelstahl oder Kunststoff.

5. Stempelinstrument (10) nach einem der vorstehenden Ansprüche, **dadurch gekennzeichnet, dass** der Schaft (16) in den Griff (20) mittels Fügetechnik, insbesondere durch Verkleben oder Umspritzen, eingebracht ist.

6. Stempelinstrument (10) nach einem der vorstehenden Ansprüche, **dadurch gekennzeichnet, dass** der Schaft (16) einen sich über die Länge des Schafts (16) erstreckenden Edelstahl-Kern sowie einen diesen umwickelnden Edelstahl-Draht aufweist.

7. Stempelinstrument (10) nach Anspruch 5, **dadurch gekennzeichnet, dass** der Edelstahl-Draht über die gesamte Länge des Edelstahlkerns gewickelt ist.

8. Stempelinstrument (10) nach einem der vorstehenden Ansprüche, **dadurch gekennzeichnet, dass** der Schaft (16) verteilt über seine Längsachse Markierungen (22) aufweist.

9. System (30) zur Behandlung von menschlichen Knochen- oder Knorpelstrukturen, insbesondere von Wirbelkörpern, zumindest umfassend das Stempelinstrument (10) nach einem der Ansprüche 1 bis 8, sowie eine erste Kanüle (32) zum Zuführen eines in der Knochen- oder Knorpelstruktur aushärtbaren Materials zu einer in der Knochen- oder Knorpelstruktur gebildeten Kavität, wobei das Stempelinstrument (10) und die erste Kanüle (32) derart ausgebildet und dimensioniert sind, dass das Stempelinstrument (10) durch die erste Kanüle (32) vorwärts bewegt werden kann, um in der Kanüle vorliegendes Material in die Kavität der Knochen-oder Knorpelstruktur zu bewegen.

10. System (30) nach Anspruch 9, **dadurch gekennzeichnet, dass** die erste Kanüle (32) einen röhrchenörmigen hohlen Körper (33) mit einem distalen (34) und einem proximalen (35) offenen Kanülenende aufweist, sowie einen einen Durchgang (37) aufweisenden Kanülengriff (36) am proximalen Kanülenende (35).

11. System (30) nach Anspruch 9 oder 10, **dadurch gekennzeichnet, dass** es ferner eine Arbeitskanüle zum Bereitstellen eines Zugangs zu einer Knochen- oder Knorpelstruktur aufweist, wobei die Arbeitskanüle derart ausgebildet und dimensioniert ist, dass die erste Kanüle (32) in die Arbeitskanüle einführbar ist.

12. System (30) nach einem der Ansprüche 9 bis 11, **dadurch gekennzeichnet, dass** es ferner ein Instrument zum Ausbilden einer Kavität in einer Knochen- oder Knorpelstruktur aufweist, welches derart ausgebildet und dimensioniert ist, dass es durch die Arbeitskanüle einführbar ist.

## Claims

1. A tamping instrument (10) for advancing through a cannula and for advancing a material present in the cannula into a cavity of a bone or cartilage structure, the tamping instrument (10) having a shaft (16), a first proximal end (12), a second distal end (14) and a handle (20) provided at the first proximal end (12), the shaft (16) being flexible, and **characterized in that** the tamping instrument (10) has a rigid tamping element (18) at its second, distal end (14), the tamping element (18) being connected to the flexible shaft (16).

2. The tamping instrument (10) according to claim 1, **characterized in that** the tamping element (18) is welded to the flexible shaft (16).

3. The tamping instrument (10) according to claim 1 or 2, **characterized in that** the shaft (16) and/or the tamping element (18) each have or consist of a material selected from the group comprising metals and non-metallic materials.

4. The tamping instrument (10) according to one of the claims 1 to 3, **characterized in that** the shaft (16) and/or the tamping element (18) each have or consist of a material selected from stainless steel or plastic.

5. The tamping instrument (10) according to one of the preceding claims, **characterized in that** the shaft (16) is joined into the handle (20) by means of a joining technique, in particular by bonding or overmolding.

6. The tamping instrument (10) according to one of the preceding claims, **characterized in that** the shaft (16) has a stainless steel core extending over the length of the shaft (16) and a stainless steel wire wrapping around the core.

7. The tamping instrument (10) according to claim 5, **characterized in that** the stainless steel wire is wound over the entire length of the stainless steel core.

8. The tamping instrument (10) according to one of the preceding claims, **characterized in that** the shaft (16) has markings (22) distributed over its longitudinal axis.

9. A system (30) for treating human bone or cartilage structures, in particular vertebral bodies, at least comprising the tamping instrument (10) according to any one of claims 1 to 8, and a first cannula (32) for supplying a material curable in the bone or cartilage structure to a cavity formed in the bone or cartilage structure, wherein the tamping instrument (10) and the first cannula (32) are configured and dimensioned such that the tamping instrument (10) can be advanced through the first cannula (32) to move material present in the cannula into the cavity of the bone or cartilage structure.

10. The system (30) according to claim 9, **characterized in that** the first cannula (32) comprises a tubular hollow body (33) having a distal (34) and a proximal (35) open cannula end, and a cannula handle (36) having a passage (37) at the proximal cannula end (35).

11. The system (30) according to claim 9 or 10, **characterized in that** it further comprises a working cannula for providing access to a bone or cartilage structure, wherein the working cannula is configured and dimensioned such that the first cannula (32) is insertable into the working cannula.

12. The system (30) of any one of claims 9 to 11, **characterized in that** it further comprises an instrument for forming a cavity in a bone or cartilage structure configured and dimensioned to be insertable through the working cannula.

## Revendications

1. Instrument à poinçon (10) destiné à avancer à travers une canule et à faire avancer un matériau présent dans la canule vers une cavité d'une structure osseuse ou cartilagineuse, l'instrument à poinçon (10) présentant une tige (16), une première extrémité proximale (12) et une deuxième extrémité distale (14), ainsi qu'une poignée (20) prévue à la première extrémité proximale (12), la tige (16) étant réalisée de manière flexible ; et
**caractérisé en ce que** l'instrument à poinçon (10) présente à sa deuxième extrémité distale (14) un élément de poinçon rigide (18) qui est relié à la tige flexible (16).

2. Instrument à poinçon (10) selon la revendication 1, **caractérisé en ce que** l'élément à poinçon (18) est soudé à la tige flexible (16).

3. Instrument à poinçon (10) selon la revendication 1 ou 2, **caractérisé en ce que** la tige (16) et/ou l'élément à poinçon (18) présentent respectivement un matériau, ou sont composés de celui-ci, qui est sélectionné dans le groupe comprenant des métaux et des matériaux non métalliques.

4. Instrument à poinçon (10) selon l'une quelconque des revendications 1 à 3, **caractérisé en ce que** la tige (16) et/ou l'élément à poinçon (18) présentent respectivement un matériau, ou sont composés de celui-ci, qui est sélectionné parmi l'acier spécial ou une matière plastique.

5. Instrument à poinçon (10) selon l'une quelconque des revendications précédentes, **caractérisé en ce que** la tige (16) est incorporée dans la poignée (20) au moyen d'une technique d'assemblage, en particulier par collage ou par surmoulage.

6. Instrument à poinçon (10) selon l'une quelconque des revendications précédentes, **caractérisé en ce que** la tige (16) présente un noyau en acier spécial s'étendant sur la longueur de la tige (16) ainsi qu'un fil d'acier spécial enrobant celui-ci.

7. Instrument à poinçon (10) selon la revendication 5, **caractérisé en ce que** le fil d'acier spécial est enroulé sur toute la longueur du noyau en acier spécial.

8. Instrument à poinçon (10) selon l'une quelconque des revendications précédentes, **caractérisé en ce que** la tige (16) présente des repères (22) répartis sur l'ensemble de son axe longitudinal.

9. Système (30) destiné à traiter des structures osseuses ou cartilagineuses humaines, en particulier des corps vertébraux, comprenant au moins l'instrument à poinçon (10) selon l'une quelconque des revendications 1 à 8 ainsi qu'une première canule (32) pour amener un matériau pouvant durcir dans la structure osseuse ou cartilagineuse à une cavité formée dans la structure osseuse ou cartilagineuse, l'instrument à poinçon (10) et la première canule (32) étant réalisés et dimensionnés de telle sorte que l'instrument à poinçon (10) peut être amené à avancer à travers la première canule (32) pour déplacer un matériau présent dans la canule vers la cavité de la structure osseuse ou cartilagineuse.

10. Système (30) selon la revendication 9, **caractérisé en ce que** la première canule (32) présente un corps creux tubulaire (33) ayant une extrémité de canule ouverte distale (34) et une extrémité proximale (35) ainsi qu'une poignée de canule (36) présentant un passage (37) à l'extrémité de canule proximale (35).

11. Système (30) selon la revendication 9 ou 10, **caractérisé en ce qu'**il présente en outre une canule de travail pour fournir un accès à une structure osseuse ou cartilagineuse, la canule de travail étant réalisée et dimensionnée de telle sorte que la première canule (32) peut être introduite dans la canule de travail.

12. Système (30) selon l'une quelconque des revendications 9 à 11, **caractérisé en ce qu'**il présente en outre un instrument pour réaliser une cavité dans une structure osseuse ou cartilagineuse, qui est réalisé et dimensionné de telle sorte qu'il peut être introduit à travers la canule de travail.
